# EUROPÄISCHE PATENTSCHRIFT

(11) **EP 3 434 327 B1**
(45) Veröffentlichungstag und Bekanntmachung des Hinweises auf die Patenterteilung: **17.06.2020**
(21) Anmeldenummer: 18185701.2
(22) Anmeldetag: 26.07.2018
(51) Int. Cl.: A61N 5/06, A61M 21/00

(54) **BESTRAHLUNGSVORRICHTUNG ZUR BESTRAHLUNG VON MENSCHLICHER HAUT**
IRRADIATION DEVICE FOR IRRADIATING HUMAN SKIN
DISPOSITIF D'IRRADIATION DESTINÉ À IRRADIER LA PEAU HUMAINE

(43) Veröffentlichungstag der Anmeldung: 30.01.2019
(73) Patentinhaber: WM Beautysystems AG & Co. KG, 50259 Pulheim (DE)
(72) Erfinder: Müller, Wolfgang, 50968 Köln (DE)
(74) Vertreter: Von Rohr Patentanwälte Partnerschaft mbB

(56) Entgegenhaltungen:
- WO-A2-2004/058352
- DE-A1-102005 003 790

## Beschreibung

Die Erfindung betrifft eine Bestrahlungsvorrichtung zur Bestrahlung von menschlicher Haut, vorzugsweise des Gesichtes eines Benutzers, mit einer Strahlungsquelle und einer Filtereinrichtung zur Filterung der von der Strahlungsquelle abgestrahlten Strahlung.

Es ist bekannt, dass bestimmte Zellen in der menschlichen Haut zur verstärkten Collagen- und Elastin-Bildung sowie zur Bildung von Enzymen angeregt werden können, die maßgeblich für die Hautstruktur verantwortlich sind. Dies wird durch den Einsatz von Geräten zur Hautpflege mittels Lichtwellen bzw. Strahlung im Bereich von 550 bis 700 nm erreicht. Dieser Prozess wird durch Anregung der Blutgefäße in der Haut erzeugt und hat eine Sauerstoffanreicherung sowie eine bessere Entgiftung zur Folge. Die Hydration nimmt zu und die Fähigkeit der Haut, Feuchtigkeit zu behalten, verbessert sich. Zunehmende Zellaktivitäten, verbesserte natürliche Zellreparatur sowie Zellerneuerungen führen zur Erzeugung eines gesünderen Hautbildes.

Im Stand der Technik sind Gesichtsbräuner bekannt, die UV-C-Strahlung in einem Wellenlängenbereich von 100 bis 280 nm zu 90 bis 100% absorbieren und UV-Strahlung im UV-A-Bereich und UV-B-Bereich (UV-B-Bereich von 280 bis 320 nm und UV-A-Bereich von 320 bis 400 nm) transmittieren. Um zusätzlich zu der Bräunung für das Gesicht des Benutzers die vorgenannten Vorteile von Strahlung im Rotlichtbereich (im Wellenlängenbereich von 600 bis 700 nm) zu erreichen, ist es bekannt, in der Nähe und/oder an der Bestrahlungsvorrichtung LED-Lampen anzuordnen, die insbesondere eine Strahlung mit einer Wellenlänge von 630 nm aussenden. So kann zusätzlich zu dem gewünschten Bräunungsergebnis der Haut die Collagen- und Elastin-Bildung der Haut angeregt werden.

Die zusätzliche Applikation der LEDs zur Bereitstellung der Strahlung im Rotlichtbereich ist jedoch mit einer Vielzahl von Nachteilen verbunden. So sind das Anbringen bzw. die Installation der LED-Strahler nicht nur aufwändig, sondern auch kostspielig bzw. mit hohen Installationskosten verbunden, insbesondere im Hinblick auf die Anpassung an die Geometrie im bzw. am Gerät sowie in der Produktion. Des Weiteren steigt der benötigte Energieverbrauch der gesamten Bestrahlungsvorrichtung inklusive der Rotlicht-LEDs drastisch an. Außerdem wird der sichtbare, insbesondere stark monochrome, Strahlungsanteil der LED-Strahler vom Benutzer als störend empfunden. Zudem kann die Strahlungsintensität und/oder Strahlungsleistung der LEDs nicht zu der Intensität der Strahlung der Bestrahlungsvorrichtung gekoppelt werden, so dass eine aufwändige Anpassung der die Strahlung im Rotlichtbereich aussendenden LEDs durchgeführt werden muss. Auch der sich mit der Zeit ergebende Abfall bzw. die Reduktion der Intensität und/oder der Leistung der LEDs und der Bestrahlungsvorrichtung ist nicht zueinander gekoppelt, so dass sich die mangelnde Anpassung der LED-Strahler zu der Bestrahlungsvorrichtung mit fortschreitender Benutzungszeit verstärkt.

Die DE 10 2005 003 790 A1 betrifft ein Bestrahlungsgerät mit einem Gehäuse, mindestens einer UV-Strahlungsquelle, die innerhalb des Gehäuses angeordnet ist, sowie einer Filterscheibe, wobei die Filterscheibe mit einer Beschichtung versehen ist. Die UV-Strahlung, sowie die sichtbare Strahlung bis 450 nm Wellenlänge tritt durch die Beschichtung hindurch, wobei Strahlung im Bereich des sichtbaren Lichts mit der Wellenlänge größer als 450 nm reflektiert wird.

Die WO 2004/058352 A2 betrifft ein Bestrahlungsgerät zur Behandlung des Krankheitsbildes Akne am Menschen, wobei das Bestrahlungsgerät Strahlungsimpulse in verschiedenen Wellenlängenbereichen aussendet.

Aufgabe der vorliegenden Erfindung ist es nun, die vorgenannten Nachteile im Stand der Technik zu vermeiden oder aber zumindest im Wesentlichen zu reduzieren.

Die vorgenannte Aufgabe wird erfindungsgemäß durch eine Bestrahlungsvorrichtung gemäß Anspruch 1 gelöst.

Die Filtereinrichtung ist derart ausgebildet ist, dass die Bestrahlungsvorrichtung Strahlung in einem Wellenlängenbereich von 280 bis 400 nm und darüber hinaus Strahlung in einem Wellenlängenbereich von 600 bis 700 nm abgibt. Die Bestrahlungsvorrichtung gibt zumindest bereichsweise Strahlung in einem Wellenlängenbereich eines sichtbaren Farbtons des sichtbaren Lichtspektrums ab und weitere Strahlung des sichtbaren Lichtspektrums filtert.

Durch die erfindungsgemäße Bestrahlungsvorrichtung ist es möglich, gleichzeitig UV-Strahlung, insbesondere UV-A-Strahlung und UV-B-Strahlung, und Strahlung im Rotlichtbereich auszusenden. Die zusätzliche Installation von Strahlung im Rotlichtbereich aussendenden LED-Strahler kann vermieden werden. Erfindungsgemäß kann das Rotlicht durch die Bestrahlungsvorrichtung selbst bereitgestellt werden. Die durch die Strahlung im Rotlichtbereich erzeugte positive Wirkung für die Haut, vorzugsweise die angeregte Collagen- und Elastin-Bildung, kann erfindungsgemäß synergetisch mit dem durch die UV-Strahlung hervorgerufenen Bräunungseffekt kombiniert werden. Bei einem Bräunen der Haut kann folglich auch die Collagen-Bildung und/oder die Elastin-Bildung der Haut angeregt werden, was zu einem verbesserten und gebräunten Hautbild der Haut führt und ein natürliches und reines Hautbild bewirkt.

Die Filtereinrichtung weist wenigstens eine Filterscheibe auf. Die Filterscheibe wiederum weist eine erste Beschichtung auf. Insbesondere ist die erste Beschichtung derart ausgebildet, dass die vorgenannten Eigenschaften der Filtereinrichtung im Hinblick auf die Filterung der Strahlung mit einer Wellenlänge von unter 280 nm sichergestellt wird.

Die erste Beschichtung ist derart ausgebildet, dass UV-A-Strahlung und UV-B-Strahlung, in einem Wellenlängenbereich von 280 bis 400 nm und Strahlung im Rotlichtbereich in einem Wellenlängenbereich von 600 bis 700 nm, vorzugsweise von 600 bis 650 nm, und Strahlung mit einer Wellenlänge von größer 700 nm transmittiert wird. Der Transmissionsgrad kann hierbei größer oder gleich 50%, bevorzugt zwischen 50 bis 100%, weiter bevorzugt zwischen 85 bis 98%, betragen. Insbesondere ist die erste Beschichtung bzw. die Filterscheibe durchlässig für die vorgenannten unterschiedlichen Wellenlängenbereiche.

Eine Transmission von Strahlung von größer als 700 nm im Infrarotbereich ist insbesondere dahingehend vorteilhaft, da Strahlung im Infrarotbereich positive Nebeneffekte bei der Bestrahlung der menschlichen Haut bewirkt, insbesondere ein angenehmes Wärmegefühl auf der Haut des Benutzers hervorruft. UV-Strahlung in einem Wellenlängenbereich von 280 bis 400 nm ruft bei der Haut des Benutzers den natürlichen Bräunungseffekt hervor.

Darüber hinaus absorbiert die erste Beschichtung UV-Strahlung im UV-C-Bereich in einem Wellenlängenbereich von 100 bis 280 nm. Hierbei kann der Absorptionsgrad größer als 0,5, bevorzugt zwischen 0,6 und 1, weiter bevorzugt zwischen 0,8 bis 0,99, betragen.

Der Absorptionsgrad gibt an, welcher Teil der Leistung einer auftreffenden Welle - elektromagnetische Strahlung, wie Licht - von einer Fläche - im vorliegenden Fall die erste Beschichtung bzw. die Filterscheibe - absorbiert, insbesondere aufgenommen, wird.

Insbesondere filtert die Filtereinrichtung UV-Strahlung im UV-C-Bereich, so dass das Bräunungsergebnis verbessert werden kann.

Vorteilhafterweise kann durch die bereichsweise Aussendung eines sichtbaren Farbtons des sichtbaren Lichtspektrums und die Filterung der weiteren Strahlung erreicht werden, dass die gesamte Bestrahlungsstärke, Strahlungsintensität und/oder Strahlungsleistung der Bestrahlungsvorrichtung zumindest bereichsweise reduziert wird, wodurch sich das Bräunungsergebnis verbessert, insbesondere sich vergleichmäßigt. Ein vorteilhafter Nebeneffekt ist hierbei, dass der ausgesendete Farbton von dem Benutzer als angenehm empfunden wird und ein Wohlbefinden auslöst.

Darüber hinaus kann durch die zumindest bereichsweise Darstellung des sichtbaren Farbtons erreicht werden, dass auf einfache Weise ein Nachweis, insbesondere eine Detektion, der durch die Bestrahlungsvorrichtung ausgesendeten Wellenlängenbereiche gelingt. Insbesondere kann der das den sichtbaren Farbton aussendende Bereich derart auf der bzw. an der Bestrahlungsvorrichtung angeordnet werden, dass empfindliche und/oder sensible Bereiche der menschlichen Haut des Benutzers, insbesondere des Gesichtes, beispielsweise der Bereich um die Augen, mit einer geringeren Bestrahlungsstärke, Strahlungsleistung und/oder Strahlungsintensität, vorzugsweise im Bereich der UV-Strahlung, bestrahlt werden. Durch den sich nach Austritt aus der Bestrahlungsvorrichtung ergebenden Strahlungskegel kann eine Überlagerung der Strahlung derart erreicht werden, dass das Bräunungsergebnis und das Hautbild ziel- und zweckgerichtet verbessert werden können.

Das sichtbare Lichtspektrum liegt in einem Wellenbereich zwischen 380 und 780 nm vor. Licht ist der Teil der elektrischen Strahlung, der für Menschen mit dem bloßen Auge sichtbar ist. Das Licht weist verschiedene Farbtöne auf, insbesondere in Abhängigkeit der Spektralfarbbereiche des Lichtes.

Letztlich versteht es sich, dass die Strahlung des Farbtons, Strahlung im Rotlichtbereich und/oder der UV-Strahlung in einem Wellenlängenbereich von 280 bis 400 nm über den gesamten Wellenbereich durch die Bestrahlungsvorrichtung ausgesendet werden kann und/oder nur einzelne Bereiche bzw. Peaks in den vorgenannten Wellenlängenbereichen vorgesehen sind. So reicht es zur Aussendung von Strahlung im Rotlichtbereich aus, wenn Strahlung in einem Wellenlängenbereich von 650 ± 20 nm ausgesendet wird. Auch eine Aussendung der UV-Strahlung in einem Wellenlängenbereich von 320 bis 400 nm wäre zur Erreichung des gewünschten Bräunungseffektes ausreichend.

Der sichtbare Farbton ist insbesondere aus den für den Menschen sichtbaren Farbbereichen ausgewählt und kann gegebenenfalls durch eine Überlagerung der Spektralfarbbereiche des Lichts erzeugt werden. Das sichtbare Lichtspektrum lässt sich in verschiedene Spektralfarbbereiche unterteilen.
Strahlung im Rotlichtbereich kann zwischen 600 bis 700 nm, insbesondere von 630 bis 700 nm, vorgesehen sein.
Strahlung im Orangelichtbereich kann zwischen 590 bis 630 nm vorgesehen sein.
Strahlung im Gelblichtbereich wiederum kann bei einem Wellenlängenbereich von 560 bis 590 nm vorgesehen sein.
Zudem kann Strahlung im Grünlichtbereich bei einem Wellenlängenbereich von 490 bis 560 nm vorgesehen sein.
Strahlung im Blaulichtbereich kann bei einer Wellenlänge zwischen 450 bis 490 nm vorgesehen sein.
Violette Strahlung bzw. Strahlung im Violettbereich kann bei einer Wellenlänge von 400 bis 450 nm vorgesehen sein.

Ein sichtbarer Farbton kann monochromatisches Licht charakterisieren. Monochromatisches Licht ist im engeren Sinn einfarbig sichtbares Licht, dessen hervorgerufene Farbwahrnehmung auch als Spektralfarbe bezeichnet wird. Die Spektralfarbbereiche des Lichtes können ineinander übergehen.

Die Bestrahlungsvorrichtung strahlt zumindest bereichsweise eine Lichtfarbe aus, wobei sich eine Lichtfarbe von einer Körperfarbe dahingehend unterscheidet, dass die Lichtfarbe die Farbe einer selbstleuchtenden Lichtquelle - im vorliegenden Fall der Strahlungsquelle - ist. Der durch die Lichtfarbe erzeugte Farbreiz beruht auf einer spektralen Zusammensetzung des Lichts. Es setzt sich entweder aus diskreten einzelnen Spektralfarben bestimmter Wellenlängen, aus einem Lichtgemisch mehrerer Wellenlängen oder Wellenlängenbereichen und/oder aus einem kontinuierlichen Spektralbereich zusammen.

Zudem kann durch die erfindungsgemäße Ausgestaltung ermöglicht werden, dass die Bestrahlungsstärke, die Strahlungsintensität und/oder die Strahlungsleistung der Strahlung im Rotlichtbereich gekoppelt zu der Strahlung im UV-Wellenlängenbereich ist.

Des Weiteren ist vorgesehen, dass die Filterscheibe eine zweite Beschichtung aufweist. Insbesondere ist die zweite Beschichtung auf der ersten Beschichtung angeordnet, und zwar weiter bevorzugt auf der der Vorderseite abgewandten Oberfläche der ersten Beschichtung. Demzufolge trifft bevorzugt die Strahlung der Strahlungsquelle zunächst auf die erste Beschichtung und anschließend auf die zweite Beschichtung.

Des Weiteren ist vorgesehen, dass die erste Beschichtung auf der der Strahlungsquelle zugewandten Vorderseite der Filterscheibe angeordnet ist. Insbesondere ist die erste Beschichtung auf der Vorderseite der Filterscheibe aufgebracht worden. Die erste Beschichtung ist als vollflächig ausgebildete Schicht auf der Filterscheibe vorgesehen, so dass die durch die erste Beschichtung hervorgerufenen Eigenschaften vollflächig über die Oberfläche der Filterscheibe sichergestellt werden.

Die zweite Beschichtung ist zur Filterung von Strahlung derart ausgebildet, dass Strahlung in wenigstens einem Wellenlängenbereich eines sichtbaren Farbtons des sichtbaren Lichtspektrums transmittiert wird und, vorzugsweise, Strahlung mit einer Wellenlänge von größer 700 nm transmittiert wird. Insbesondere wird weitere Strahlung, vorzugsweise weitere Strahlung des sichtbaren Lichtspektrums, ansonsten durch die zweite Beschichtung reflektiert und/oder absorbiert. Der Wellenlängenbereich eines sichtbaren Farbtons kann insbesondere Strahlung im Grünlicht-, Rotlicht- und/oder Gelblichtbereich sein. Letztlich wird zumindest ein solcher Wellenlängenbereich über die zweite Beschichtung transmittiert, der eine für einen Benutzer wahrnehmbare Farbwirkung bedingt.

Des Weiteren ist die zweite Beschichtung als teilflächig ausgebildete Schicht ausgebildet. Insbesondere ist die zweite Beschichtung unterbrochen. Vorzugsweise kann durch die zweite Beschichtung erreicht werden, dass die Bestrahlungseinrichtung dazu eingerichtet ist, zumindest bereichsweise Strahlung in einem Wellenlängenbereich eines sichtbaren Farbtons des sichtbaren Lichtspektrums abzugeben und weitere Strahlung des sichtbaren Lichtspektrums zu filtern.

Demzufolge sind weiter bevorzugt auf der Filterscheibe Bereiche, die sowohl mit der ersten Beschichtung als auch mit der zweiten Beschichtung versehen sind, und weitere Bereiche, die lediglich die erste Beschichtung aufweisen, vorgesehen. Letztlich versteht es sich, dass die Filterscheibe wenigstens die durch die erste Beschichtung zur Verfügung gestellten Eigenschaften aufweist, insbesondere dann, wenn die erste Beschichtung vollflächig auf die Filterscheibe aufgebracht worden ist. In den mit der zweiten Beschichtung versehenen Bereichen können - übergelagert zu den Eigenschaften der ersten Beschichtung - weitere Wellenlängenbereich gefiltert werden.

Bei einer besonders bevorzugten Ausführungsform ist vorgesehen, dass die Filtereinrichtung derart ausgebildet ist, dass sie Strahlung mit einer Wellenlänge von unter 280 nm filtert. Insbesondere kann vorgesehen sein, dass eine Filterung durch Reduktion der Strahlungsintensität größer als 60%, bevorzugt zwischen 70 und 100%, weiter bevorzugt zwischen 90 und 99,99%, für Wellenlängen von unter 280 nm erfolgt. Eine Filterung der vorgenannten Wellenlängenbereiche der Strahlung kann zu einer Verringerung der Bestrahlungsstärke, der Strahlungsleistung und/oder der Strahlungsintensität führen. Besonders bevorzugt ist die Filterung derart ausgebildet, dass zumindest im Wesentlichen keine Strahlung mit einer Wellenlänge von unter 280 nm durch die Bestrahlungsvorrichtung ausgesendet wird.

Die vorgenannte Filterung der Strahlung für Wellenlängen von unter 280 nm ist insbesondere dahingehend vorteilhaft, da Strahlung mit einer Wellenlänge von unter 280 nm, keine vorteilhafte Wirkung zur Verbesserung des Hautbildes zeigt und sogar schädlich für den Benutzer, insbesondere die Haut des Benutzers, sein kann. Demgemäß wird vorzugsweise die nicht zur Bräunung des Gesichtes beitragende Strahlung mittels der Filtereinrichtung gefiltert.

Insbesondere ist die Filterscheibe derart in der Bestrahlungsvorrichtung angeordnet, dass die gesamte Strahlung der Strahlungsquelle, die die Bestrahlungsvorrichtung verlässt, die Filterscheibe passieren muss, insbesondere wobei die Strahlung an der Filterscheibe transmittiert, absorbiert und/oder reflektiert wird.

Als Transmission ist die Größe für die Durchlässigkeit eines Mediums - im vorliegenden Fall der Bestrahlungsvorrichtung und/oder der Filterscheibe - für elektromagnetische Wellen, insbesondere Licht, zu verstehen. Der Transmissionsgrad ist eine Materialeigenschaft und ist anhand des Quotienten zwischen der Wellenintensität hinter und vor dem Hindernis definiert. Der Transmissionsgrad ist demgemäß ein Maß für die durchgelassene Intensität und kann zwischen 0 und 1 bzw. zwischen 0% bis 100% variieren.

Bei einer weiteren ganz besonders bevorzugten Ausführungsform des Erfindungsgedankens ist die erste Beschichtung, derart ausgebildet, dass UV-B-Strahlung in einem Wellenlängenbereich von 280 bis 320 nm mit einem Transmissionsgrad von kleiner oder gleich 50%, bevorzugt zwischen 0,1 bis 50%, weiter bevorzugt zwischen 1 bis 8%, transmittiert wird. Insbesondere ist der transmittierte Anteil der UV-B-Strahlung an gesetzliche Vorgaben angepasst.

Zusätzlich oder alternativ kann die erste Beschichtung derart ausgebildet sein, dass UV-Strahlung in einem Wellenlängenbereich von 320 bis 400 nm mit einem Transmissionsgrad von größer oder gleich 40%, bevorzugt zwischen 45 bis 95%, weiter bevorzugt zwischen 50% bis 70%, transmittiert wird.

Ferner kann die erste Beschichtung derart ausgebildet sein, dass Strahlung im Blaulichtbereich in einem Wellenlängenbereich von 400 bis 480 nm mit einem Transmissionsgrad von größer gleich 20%, bevorzugt zwischen 30 bis 95%, weiter bevorzugt zwischen 60 und 80%, transmittiert wird. Bei der gleichzeitigen Transmission von Strahlung im Rotlichtbereich über die erste Beschichtung kann eine Überlagerung mit der Strahlung im Blaulichtbereich erfolgen, so dass sich eine veränderte Farbwahrnehmung für den Benutzer ergibt.

Vorzugsweise ist die zweite Beschichtung fest mit der ersten Beschichtung verbunden. Eine Verbindung kann unmittelbar oder mittelbar erfolgen. Bei einem mittelbaren Verbund ist vorgesehen, dass zwischen der ersten Beschichtung und der zweiten Beschichtung wenigstens eine weitere Lage und/oder Schicht der Filterscheibe vorgesehen ist.

Darüber hinaus kann die zweite Beschichtung auf der Rückseite der Filterscheibe, der Bestrahlungsquelle abgewandt, angeordnet sein, insbesondere wobei die zweite Beschichtung auf der Rückseite aufgebracht worden ist.

Die zweite Beschichtung kann derart ausgebildet sein, dass auf der Rückseite der Filterscheibe - für den Benutzer sichtbar - ein Farbton dargestellt wird bzw. eine sichtbare Farbwirkung eines sichtbaren Farbtons hervorgerufen wird.

Außerdem kann die zweite Beschichtung rasterförmig, regelmäßig und/oder unregelmäßig auf der Filterscheibe aufgebracht worden bzw. vorgesehen sein. Die mit der zweiten Beschichtung versehenen Bereiche der Filterscheibe sind derart ausgewählt, insbesondere im Hinblick auf die mit der zweiten Beschichtung bedeckten Oberflächenbereiche der Fläche, dass verbesserte Strahlungsergebnisse der mit der Bestrahlungsvorrichtung bestrahlten menschlichen Haut erreicht werden können.

Vorzugsweise kann die zweite Beschichtung über eine Schablone auf die Rückseite der Filterscheibe und/oder auf die erste Beschichtung aufgebracht werden. Die Schlablone kann als Material Glas und/oder Metall und/oder ein steinbasiertes und/oder hitzebeständiges Material aufweisen. Die Freiflächen ermöglichen die bereichsweise Bechichtung der Filterscheibe mit der zweiten Beschichtung.

Verfahrensgemäß kann beim Beschichten der Filterscheibe mit der ersten und/oder der zweiten Beschichtung vorgesehen sein, dass eine Beschichtung im Vakuum, vorzugsweise Hochvakuum durchgeführt wird. Ein Beschichten kann mit dielektrischen Schichten bzw. Beschichtungen erfolgen. Die dielektrischen Schichten der ersten und/oder der zweiten Beschichtung ermöglichen insbesondere das gewünschte Transmissions-, Absorptions- und/oder Reflektionsverhalten.

In den während des Zustandekommens der Erfindung durchgeführten Versuchen konnte gezeigt werden, dass durch ein Zusammenspiel der ersten Beschichtung und der zweiten Beschichtung verbesserte Bräunungsergebnisse bei einer gleichzeitig vorhandenen Collagen- und Elastinbildung, die eine natürliche Bräunung bewirken, sichergestellt werden können.

Durch das bereichsweise Aufbringen der zweiten Beschichtung können gezielt einzelne Wellenlängenbereiche und/oder monochromatisches Licht für den Benutzer optisch sichtbar gemacht werden, so dass eine Kontrollfunktion und/oder Detektier-Funktion der über die Bestrahlungsvorrichtung ausgesendeten Bestrahlung erfolgt.

Darüber hinaus kann auf der Filterscheibe eine Vielzahl von unterschiedlichen zweiten Beschichtungen vorgesehen sein, die sich hinsichtlich ihrer Filterung, Geometrie, Schichtdicke und/oder ihrer Eigenschaften, insbesondere Materialeigenschaften, unterscheiden. So können verschiedene sichtbare Farbtöne auf der Rückseite der Filterscheibe, die den Benutzer zugewandt ist, hervorgerufen werden.

Alternativ oder zusätzlich kann vorgesehen sein, dass die zweite Beschichtung derart ausgebildet ist, dass UV-Strahlung in einem Wellenlängenbereich von 100 bis 400 nm und/oder Blaulicht in einem Wellenlängenbereich von 400 bis 480 nm mit einem Transmissionsgrad von kleiner oder gleich 20%, bevorzugt zwischen 0,01 bis 50%, weiter bevorzugt zwischen 0,1 bis 5%, transmittiert wird. Insbesondere wird UV-Strahlung und/oder Blaulicht durch die zweite Beschichtung gefiltert, so dass Strahlung, die durch die zweite Beschichtung transmittiert wird, vorzugsweise nur einen sehr geringen Anteil an Blaulicht und/oder UV-Strahlung aufweist.

Ferner ist die zweite Beschichtung vorzugsweise derart ausgebildet, dass Strahlung im Rotlichtbereich in einem Wellenlängenbereich von 600 bis 700 nm und/oder Strahlung mit einer Wellenlänge von größer als 700 nm mit einem Transmissionsgrad von größer oder gleich 50%, bevorzugt zwischen 50 bis 100%, weiter bevorzugt zwischen 75 bis 85%, transmittiert wird. Vorzugsweise transmittiert die zweite Beschichtung Strahlung im Rotlichtbereich und Strahlung mit einer Wellenlänge von größer als 700 nm im Infrarotbereich. Für den Benutzer, der der Rückseite der Filterscheibe zugewandt ist, können die die zweite Beschichtung aufweisenden Bereiche der Filterscheibe insbesondere rot wirken bzw. Rotlicht aussenden. Hierdurch kann eindeutig identifiziert bzw. erkannt werden, dass auch über die erste Beschichtung - also in denjenigen Bereichen, die nicht mit der zweiten Beschichtung versehen sind - Rotlicht transmittiert wird. Diejenigen Anteile der Strahlung, die bereits durch die erste Beschichtung gefiltert worden sind, werden insbesondere durch die zweite Beschichtung weiter reduziert, insbesondere gefiltert, und/oder transmittiert.

Gemäß einer weiteren Ausführungsform kann vorgesehen sein, dass die zweite Beschichtung UV-Strahlung in einem Wellenlängenbereich von 320 bis 400 nm mit einem Transmissionsgrad von größer oder gleich 40%, bevorzugt zwischen 45 bis 95%, weiter bevorzugt zwischen 50 bis 70%, transmittiert. Vorzugsweise transmittiert die zweite Beschichtung Strahlung im Rotlichtbereich, Strahlung mit einer Wellenlänge von über 700 nm und Strahlung im Bereich von 320 bis 400 nm und/oder Strahlung im UV-A und/oder UV-B-Bereich.

Zudem ist die UV-Strahlung nicht für den der Rückseite der Filterscheibe zugewandten Benutzer mit bloßem Auge optisch sichtbar.

Von besonderem Vorteil im Zusammenhang mit der vorliegenden Erfindung ist es, wenn die Strahlungsquelle in einer Reflektoreinrichtung vorgesehen ist, der eine Kühleinrichtung zugeordnet ist. Dabei weist die Reflektoreinrichtung einen Reflektor auf, in dem die Strahlungsquelle, vorzugsweise eine Bestrahlungslampe, angeordnet ist. Die Filtereinrichtung kann der Reflektoreinrichtung zugeordnet sein und/oder die Reflektoreinrichtung weist die Filtereinrichtung auf.

Der Vorteil der vorliegenden Erfindung besteht darin, dass die erfindungsgemäße Reflektoreinrichtung in Form und Größe grundsätzlich einer Reflektoreinrichtung entsprechen kann, die beim Stand der Technik bei Bestrahlungsvorrichtungen zur Bestrahlung des Gesichtes des Benutzers eingesetzt wird. Die bekannte Reflektoreinrichtung zur Gesichtsbräunung kann durch die erfindungsgemäße Reflektoreinrichtung ohne weiteres ausgetauscht werden und/oder die erfindungsgemäße Filtereinrichtung kann ohne weiteres in die bereits vorhandene Reflektoreinrichtung integriert und/oder an dieser angeordnet werden. Dies ermöglicht es, bekannte Gesichtsbräuner erfindungsgemäß umzurüsten.

Zudem kann auch die Reflektoreinrichtung und/oder der Reflektor selbst zur Filtereinrichtung gehören und insbesondere derart ausgebildet sein, dass die Reflektoreinrichtung eine Strahlung im Wellenlängenbereich von 100 bis 480 nm, vorzugsweise in einem Wellenlängenbereich von 280 bis 400 nm, und/oder in einem Wellenlängenbereich von 600 bis 700 nm und/oder im Wellenlängenbereich von größer 700 nm reflektiert, insbesondere aussendet. Vorzugsweise beträgt hierbei der Reflektionsgrad größer oder gleich 40%, bevorzugt zwischen 45 und 95%, weiter bevorzugt zwischen 50 bis 70%. Des Weiteren kann vorgesehen sein, dass die Reflektoreinrichtung und/oder der Reflektor ansonsten transmittiert und/oder absorbiert, insbesondere filtert und/oder blockt. Hierdurch wird dann der gewünschte Wellenlängenbereich bereits durch den Reflektor und/oder die Reflektoreinrichtung entsprechend gefiltert.

Der Reflektor der Reflektoreinrichtung kann insbesondere beschichtet sein, vorzugsweise mit der ersten und/oder zweiten Beschichtung. So können nur diejenigen Wellenlängenbereiche der Strahlung abgegeben werden, die erwünscht sind.

Ferner kann die Reflektoreinrichtung ein Reflektionsmittel aufweisen, das derart ausgebildet ist, dass die von der Strahlungsquelle ausgesendete Strahlung zumindest im Wesentlichen vollumfänglich auf den Reflektor trifft.

Vorzugsweise ist die Strahlungsquelle als wenigstens eine Bestrahlungslampe ausgebildet, insbesondere als Hochdruck- und/oder Höchstdruck-Gasentladungslampe.

Insbesondere kann ein regelbares oder nicht regelbares, insbesondere magnetisches oder elektronisches, Vorschaltgerät vorgesehen sein. Das Vorschaltgerät kann vorzugsweise die Strahlungsquelle, insbesondere die Bestrahlungslampe, dimmen bzw. die Strahlungsquelle ist aufgrund des Vorschaltgerätes dimmbar.

Im Übrigen versteht es sich, dass in den vorgenannten Intervallen und Bereichsgrenzen jegliche Zwischenintervalle und Einzelwerte enthalten und als erfindungswesentlich offenbart anzusehen sind, selbst wenn diese Zwischenintervalle und Einzelwerte nicht konkret angegeben sind.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Die Erfindung ist in den Ansprüchen definiert; die anderen Ausführungsbeispiele sind lediglich als illustrativ zu verstehen.

Es zeigt:
- Fig. 1: eine schematische Vorderansicht einer erfindungsgemäßen Bestrahlungsvorrichtung,
- Fig. 2: eine schematische perspektivische Darstellung einer weiteren Ausführungsform einer erfindungsgemäßen Bestrahlungsvorrichtung,
- Fig. 3: eine schematische Ansicht von Teilen einer Bestrahlungsvorrichtung gemäß einer ersten Ausführungsform,
- Fig. 4: eine schematische Ansicht von Teilen einer Bestrahlungsvorrichtung gemäß einer zweiten Ausführungsform,
- Fig. 5: eine schematische Ansicht von Teilen einer Bestrahlungsvorrichtung gemäß einer dritten Ausführungsform,
- Fig. 6a: eine schematische perspektivische Darstellung einer erfindungsgemäßen Filterscheibe,
- Fig. 6b: schematische Querschnittsansicht entlang des Schnitts A-A aus Fig. 6a,
- Fig. 7: eine Darstellung der Transmission einer erfindungsgemäßen Bestrahlungsvorrichtung über die Wellenlänge,
- Fig. 8: eine schematische Ansicht einer erfindungsgemäßen Reflektoreinrichtung,
- Fig. 9: eine schematische Darstellung einer weiteren Ausführungsform einer nicht erfindungsgemäßen Bestrahlungsvorrichtung,
- Fig. 10: eine schematische perspektivische Darstellung einer weiteren Ausführungsform einer erfindungsgemäßen Bestrahlungsvorrichtung und
- Fig. 11: eine schematische perspektivische Darstellung einer weiteren Ausführungsform einer erfindungsgemäßen Bestrahlungsvorrichtung.

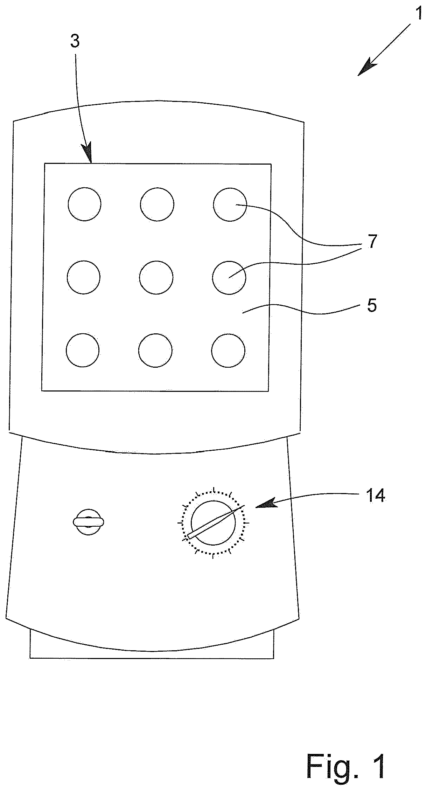

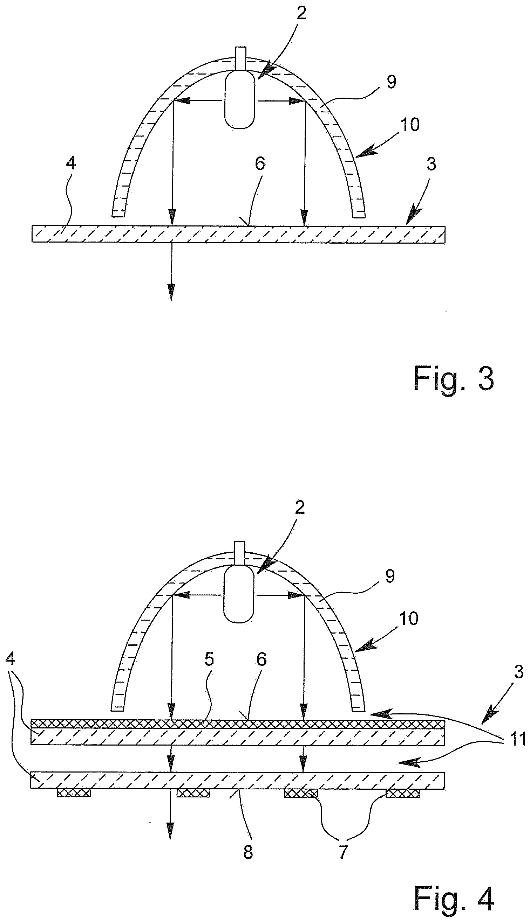

Fig. 1 zeigt eine Bestrahlungsvorrichtung 1 zur Bestrahlung von menschlicher Haut mit einer Strahlungsquelle 2, wie in Fig. 3 ersichtlich, und mit einer Filtereinrichtung 3 zur Filterung der von der Strahlungsquelle 2 abgestrahlten Strahlung.

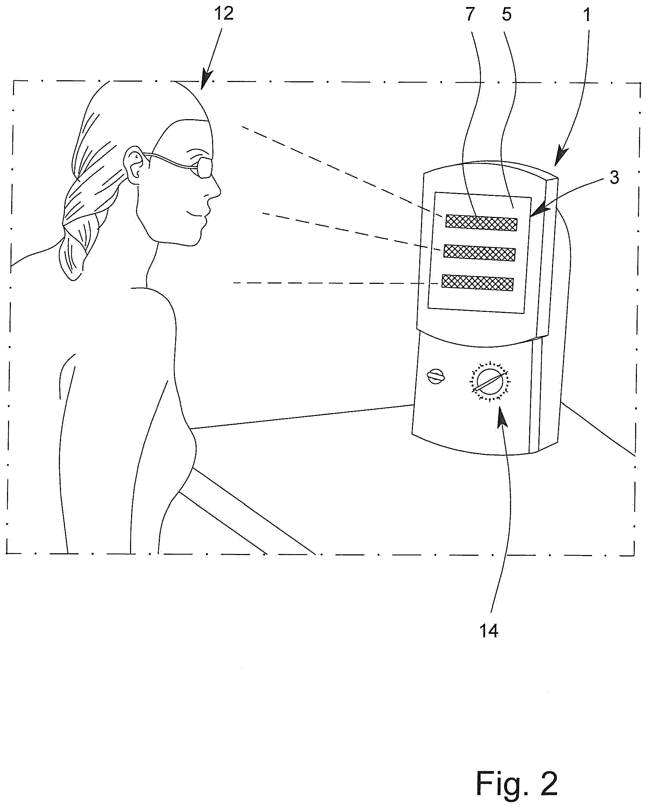

Fig. 2 zeigt, dass die Bestrahlungsvorrichtung 1 zur Bestrahlung des Gesichtes eines Benutzers 12 vorgesehen ist. Die Bestrahlungsvorrichtung 1 sendet Strahlung im Rotlichtbereich - das heißt Rotlicht in einem Wellenlängenbereich von 600 bis 700 nm - aus. Die in den dargestellten Ausführungsformen gezeigte Filtereinrichtung 3 ist derart ausgebildet, dass die Bestrahlungsvorrichtung 1 Strahlung in einem Wellenlängenbereich von 280 bis 400 nm und Strahlung in einem Wellenlängenbereich von 600 bis 700 nm abgibt und dass die Bestrahlungsvorrichtung 1 zumindest bereichsweise Strahlung in einem Wellenlängenbereich eines sichtbaren Farbtons des sichtbaren Lichtspektrums abgibt und weitere Strahlung des sichtbaren Lichtspektrums filtert. Dass auf der dem Benutzer 12 zugewandten Seite der Bestrahlungsvorrichtung 1 Bereiche vorgesehen sind, die Strahlung in einem Farbton transmittieren und weitere Strahlung des sichtbaren Lichtspektrums filtern, ist in den Fig. 1 und 2 dargestellt.

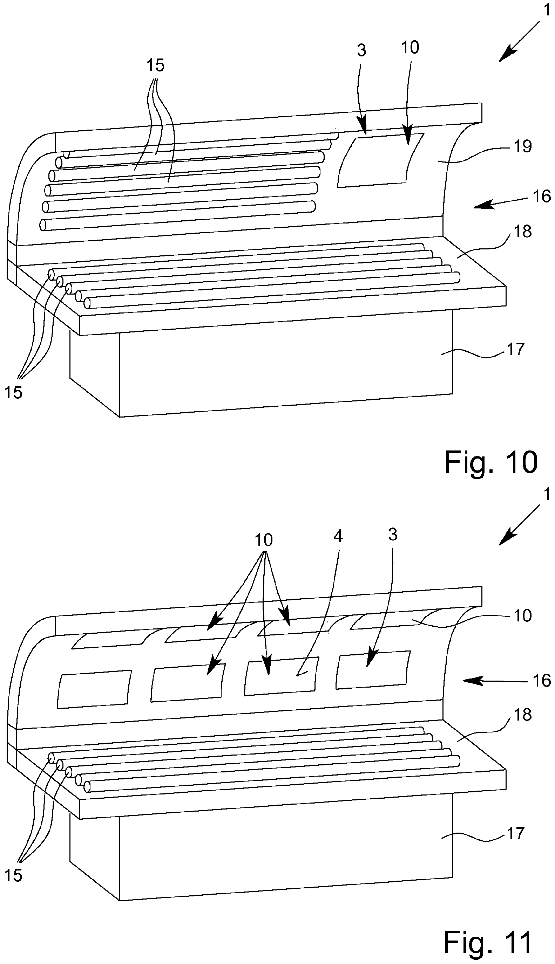

In Fig. 10 ist eine als Sonnen- oder Bestrahlungsbank ausgebildete Bestrahlungsvorrichtung 1 zur Bestrahlung menschlicher Haut dargestellt. Bei der Bestrahlungsvorrichtung 1 handelt es sich um ein sogenanntes Tunnelgerät, das einen Unterbau 17 und ein am Unterbau 17 abgelenktes, verschwenkbares Oberteil 19 aufweist. Das Oberteil 19 ist auf den Unterbau 17 herabschwenkbar, so dass sich ein Tunnel ergibt, in dem sich während des Betriebes der Benutzer 12 befindet. Unterhalb der Liegefläche 18 im Unterbau 17 und im Oberteil 19 befinden sich vorliegend langgestreckte Niederdruck-Leuchtstoff-Lampen 15, die auch Strahlung im Rotlichtbereich abgeben können.

Im Gesichtsbereich 16 des Oberteils 19 der Bestrahlungsvorrichtung 1 befindet sich eine Filtereinrichtung 3 mit einer Filterscheibe 4 angeordnet an einer Reflektoreinrichtung 10.

In Fig. 11 ist eine Ausführungsform dargestellt, bei der im Bereich des Oberteils 19 nicht nur eine, sondern eine Mehrzahl von Reflektoreinrichtungen 10 bzw. von Filtereinrichtungen 3 einer nicht dargestellten Strahlungsquelle 2 vorgesehen ist. Im dargestellten Ausführungsbeispiel sind acht Reflektoreinrichtungen 10 und Filtereinrichtungen 3 vorgesehen, die paarweise angeordnet sind.

Nicht dargestellt ist, dass auch im Unterbau 17 wenigstens eine Strahlungsquelle 2 mit zugehöriger Filtereinrichtung 3 vorgesehen ist.

Die Filtereinrichtung 3 ist gemäß der in Fig. 3 gezeigten Ausführungsform derart ausgebildet, dass sie Strahlung mit einer Wellenlänge von unter 100 nm filtert. Eine Filterung bedingt eine Reduktion der Bestrahlungsstärke, der Strahlungsleistung und/oder der Strahlungsintensität, insbesondere im Bereich zwischen 90 und 100%.

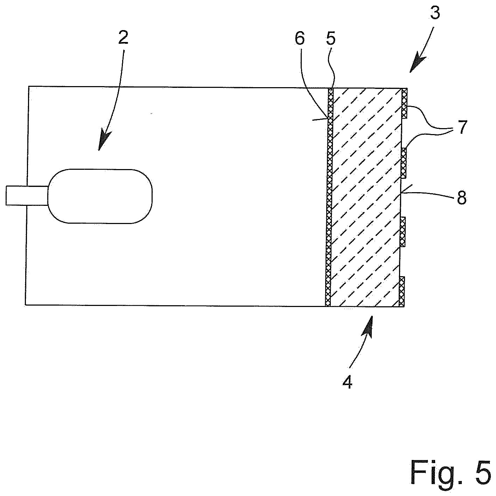

In Fig. 4 ist gezeigt, dass die Filtereinrichtung 3 eine Filterscheibe 4 aufweist. Die Filterscheibe 4 kann wiederum gemäß dem in Fig. 4 und 5 dargestellten Ausführungsbeispiel auf der der Strahlungsquelle 2 zugewandten Vorderseite 6 der Filterscheibe 4 angeordnet sein, vorzugweise aufgebracht worden sein. Die erste Beschichtung 5 kann als vollflächig ausgebildete Schicht, wie Fig. 5 verdeutlicht, vorgesehen sein.

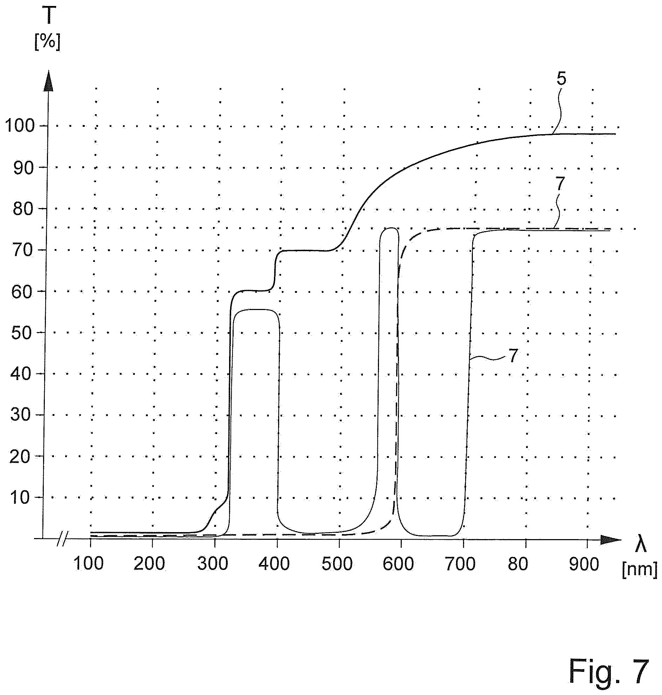

In Fig. 7 ist die Transmission der ersten Beschichtung 5 für verschiedenen Wellenlängenbereiche dargestellt. Lambda bzw. die x-Achse gibt die Wellenlänge in nm an, wobei die y-Achse die Transmission bzw. den Transmissionsgrad in Prozent wiedergibt. Gemäß dem in Fig. 7 dargestellten Verhalten ist vorgesehen, dass die erste Beschichtung 5 derart ausgebildet ist, dass die Transmission der UV-Strahlung in einem Wellenlängenbereich von 280 bis 400 nm - also UV-A-Strahlung und UV-B-Strahlung - größer gleich 50% beträgt. Der Transmissionsgrad von UV-Strahlung mit einer Wellenlänge zwischen 280 bis 320 nm beträgt letztlich gemäß dem in Fig. 7 dargestellten Verhalten zwischen 1 bis 8%, im Durchschnitt etwa 5% ± 3%. UV-Strahlung in einem Wellenlängenbereich von 100 bis 280 nm wird absorbiert, wobei der Absorptionsgrad gemäß dem in Fig. 7 dargestellten Verhalten 0,8 bis 0,99 beträgt.

Des Weiteren ist die erste Beschichtung 5 derart ausgebildet, dass Strahlung im Rotlichtbereich - also Strahlung mit einer Wellenlänge von größer 600 bis 700 nm - mit einem Transmissionsgrad von etwa 95% ± 5% transmittiert bzw. abgestrahlt wird. Ferner wird auch Strahlung mit Wellenlängen von größer als 700 nm transmittiert und vorzugsweise nicht gefiltert, so dass der Transmissionsgrad für Strahlung mit einer Wellenlänge von über 700 nm auch 95% ± 5% betragen kann.

Außerdem kann auch vorgesehen sein, dass die erste Beschichtung 5 derart ausgebildet ist, dass UV-Strahlung in einem Wellenlängenbereich von 280 bis 400 nm und/oder Strahlung im Rotlichtbereich in einem Wellenlängenbereich von 600 bis 700 nm, vorzugsweise von 600 bis 650 nm, und/oder Strahlung mit einer Wellenlänge von größer als 700 nm transmittiert wird. Der Transmissionsgrad kann hierbei größer gleich 50%, weiter bevorzugt zwischen 85% bis 95%, betragen.

Wie zuvor dargelegt, zeigt Fig. 7, dass die erste Beschichtung 5 derart ausgebildet ist, dass UV-B-Strahlung in einem Wellenlängenbereich von 280 bis 320 nm mit einem Transmissionsgrad von kleiner gleich 50%, bevorzugt zwischen 1% und 8%, transmittiert wird.

Außerdem ist die Filterscheibe 4, insbesondere die erste Beschichtung 5, derart ausgebildet, dass UV-Strahlung in einem Wellenlängenbereich von 320 bis 400 nm und einem Transmissionsgrad von größer oder gleich 40%, bevorzugt zwischen 50% bis 70%, transmittiert wird.

Darüber hinaus geht aus dem in Fig. 7 dargestellten Verhalten hervor, dass die Filterscheibe 4, insbesondere die erste Beschichtung 5, derart ausgebildet ist, dass Strahlung im Blaulichtbereich in einem Wellenlängenbereich von 400 bis 480 nm mit einem Transmissionsgrad von größer oder gleich 20%, bevorzugt zwischen 60% und 80%, transmittiert wird. Letztlich versteht es sich, dass das in Fig. 7 dargestellte Verhalten der Transmission der ersten Beschichtung 5 auch dem gesamten Abstrahlungsverhalten der Bestrahlungsvorrichtung 1 entsprechen kann.

Fig. 4 zeigt, dass die Filterscheibe 4 eine zweite Beschichtung 7 aufweist. Bei der in Fig. 4 dargestellten Ausführungsform sind letztlich zwei Filterscheiben 4 vorgesehen, wobei die erste, der Strahlungsquelle 2 zugewandte, Filterscheibe 4 an ihrer Vorderseite 6 eine erste Beschichtung 5 aufweist. Die zweite Filterscheibe weist auf der der Strahlungsquelle 2 abgewandten Rückseite 8 zumindest bereichsweise eine zweite Beschichtung 7 auf. Zwischen den Filterscheiben 4 ist eine Kühleinrichtung 11 bzw. ein Luftspalt vorgesehen.

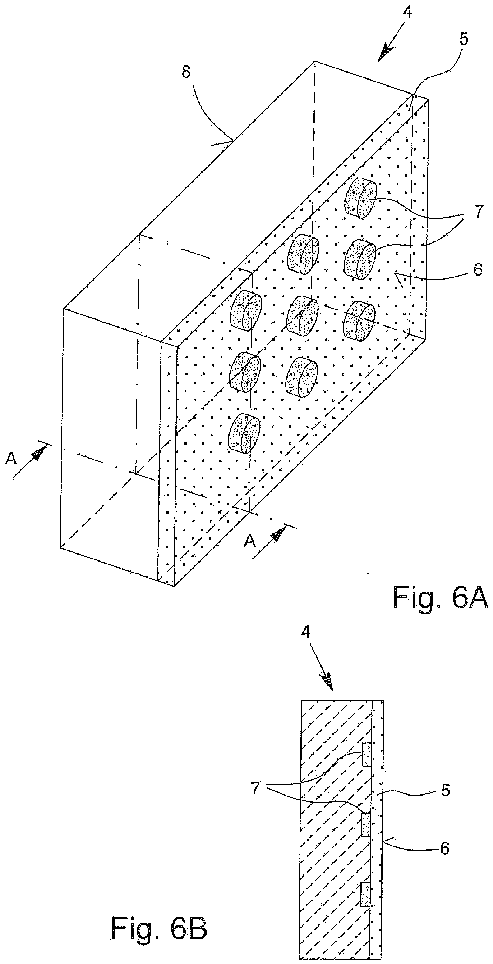

Bei der in Fig. 6a und 6b gezeigten Ausführungsform ist die zweite Beschichtung 7 auf der ersten Beschichtung 5 angeordnet, wobei die zweite Beschichtung 7 fest mit der ersten Beschichtung 5 verbunden ist.

Fig. 5 zeigt, dass die zweite Beschichtung 7 auf der Rückseite 8 der Filterscheibe 4, der Strahlungsquelle 2 abgewandt, angeordnet ist, insbesondere aufgebracht ist, wobei die erste Beschichtung 5 auf der Vorderseite 6 der Filterscheibe 4 vorgesehen ist.

Die in Fig. 5 bis 6b gezeigte zweite Beschichtung 7 ist als teilflächig ausgebildete Schicht ausgebildet. Die teilflächige zweite Beschichtung 7 ist demzufolge eine unterbrochene Schicht. Letztlich können mehrere unterbrochene bzw. teilflächige zweite Beschichtungen 7 auf der Filterscheibe 4 vorgesehen sein, die sich hinsichtlich ihrer Materialeigenschaften und/oder Funktionen unterscheiden.

Die zweite Beschichtung 7 ist zur Filterung der Strahlung ausgebildet, wobei Strahlung in wenigstens einem Wellenlängenbereich eines sichtbaren Farbtons des sichtbaren Lichtspektrums transmittiert wird. Als sichtbarer Farbton kann eine Strahlung im Grünlicht-Wellenlängenbereich, Rotlicht-Wellenlängenbereich und/oder Gelblicht-Wellenlängenbereich vorgesehen sein. Der Grünlichtbereich ist insbesondere bei einer Wellenlänge von 490 bis 560 nm, der Gelblichtbereich von 560 bis 590 nm und der Rotlichtbereich von 600 bis 700 nm, bevorzugt von 630 bis 700 nm, vorgesehen. Zudem kann die zweite Beschichtung 7 Strahlung mit einer Wellenlänge von größer 700 nm transmittieren, insbesondere wobei ansonsten die zweite Beschichtung 7 reflektiert und/oder absorbiert.

Die gesamte Bestrahlungsvorrichtung 1 kann demzufolge in über diejenigen Bereiche der Filterscheibe 4, die nur die erste Beschichtung 5 aufweisen, die gefilterte Strahlung durch die erste Beschichtung 5 transmittieren. In denjenigen Bereichen, in denen zusätzlich oder alternativ zu der ersten Beschichtung 5 die zweite Beschichtung 7 vorgesehen ist, wird die Strahlung insbesondere zum einen durch die erste Beschichtung 5 und zum anderen durch die zweite Beschichtung 7 gefiltert, so dass die zweite Beschichtung 7 die erste Beschichtung 5 überlagert.

Fig. 7 zeigt, dass die zweite Beschichtung 7, insbesondere in Kombination mit der ersten Beschichtung 5 und/oder überlagert durch die erste Beschichtung 5 transmittierte Strahlung, derart ausgebildet ist, dass UV-Strahlung in einem Wellenlängenbereich von 100 bis 400 nm und Strahlung im Blaulichtbereich in einem Wellenlängenbereich von 400 bis 480 nm mit einem Transmissionsgrad von kleiner als 20%, im dargestellten Ausführungsbeispiel mit einem Transmissionsgrad von 1,5% + 5% ± 0,5% transmittiert wird.

Die in Fig. 7 dargestellte Transmission der zweiten Beschichtung 7 kann der gesamten Transmission der durch die Bestrahlungsvorrichtung 1 abgegebenen Strahlung entsprechen, wobei die in der Bestrahlungsvorrichtung 1 eingesetzte Filterscheibe 4 eine vollflächige erste Beschichtung 5 und bereichsweise die zweite Beschichtung 7 aufweisen kann.

Das Transmissionsverhalten einer alternativen zweiten Beschichtung 7 ist auch in der Fig. 7 dargestellt, wobei diese zweite Beschichtung 7 auch UV-Strahlung in einem Wellenlängenbereich von 320 bis 400 nm mit einem Transmissionsgrad von größer als 40% transmittiert. In dem dargestellten Ausführungsbeispiel ist ein Transmissionsgrad von 55% ± 5% vorgesehen. Des Weiteren transmittieren beide zweiten Beschichtungen 7, die in Fig. 7 dargestellt sind, Strahlung mit einer Wellenlänge von größer als 700 nm mit einem Transmissionsgrad von 75% ± 5%.

Des Weiteren kann die zweite Beschichtung 7 derart ausgebildet sein, dass Strahlung im Rotlichtbereich in einem Wellenlängenbereich von 600 bis 700 nm transmittiert wird, und zwar bei einem Transmissionsgrad von größer gleich 50%. Bei dem in Fig. 7 dargestellten Ausführungsbeispiel ist ein Transmissionsgrad für Strahlung im Rotlichtbereich von 75% ± 5% vorgesehen.

Bei einer weiteren in Fig. 7 dargestellten Ausführungsform der zweiten Beschichtung 7 ist vorgesehen, dass anstelle von Strahlung im Rotlichtbereich ein anderer sichtbarer Farbton als "Rot" dargestellt werden kann. Gemäß dem in Fig. 7 dargestellten Ausführungsbeispiel wird Strahlung im Gelblichtbereich bei einem Wellenlängenbereich von 560 bis 590 nm mit einem Transmissionsgrad von 75 ± 5% über die zweite Beschichtung 7 - gegebenenfalls überlagert durch die erste Beschichtung 5 - transmittiert.

Die Fig. 3 und 4 zeigen, dass wenigstens eine einen Reflektor 9 aufweisende Reflektoreinrichtung 10 vorgesehen ist. Gemäß dem in Fig. 4 dargestellten Ausführungsbeispiel ist eine der Reflektoreinrichtung 10 zugeordnete Kühleinrichtung 11 vorgesehen. Die Kühleinrichtung 11 umfasst im dargestellten Ausführungsbeispiel zwei Luftspalte zwischen den Filterscheiben 4. Die Strahlungsquelle 2 ist im Bereich des Reflektors 9 angeordnet.

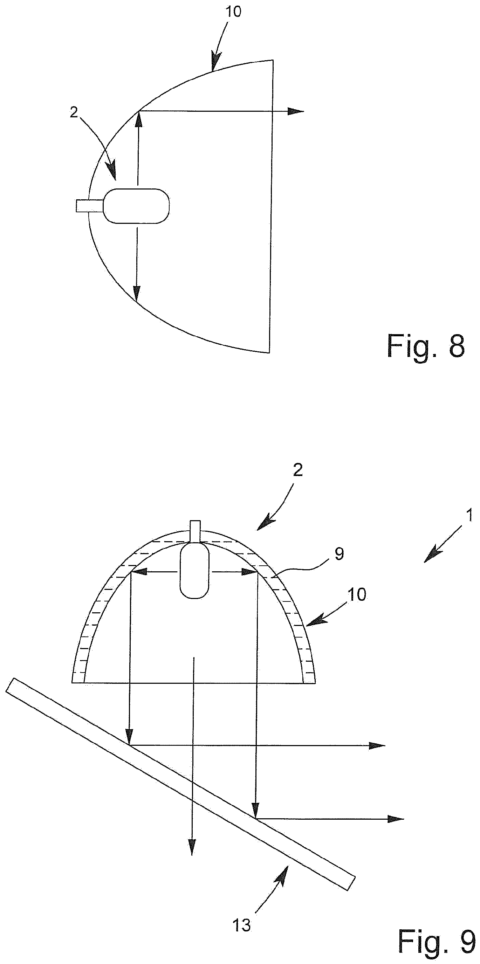

Fig. 9 zeigt, dass die Filtereinrichtung 3 einen schräggestellten Filterspiegel 13 aufweist. Der in Fig. 9 dargestellte Filterspiegel 13 ist derart ausgebildet, dass Strahlung im Wellenlängenbereich von 280 bis 400 nm - das heißt Strahlung im UV-Bereich, vorzugsweise von 280 bis 320 nm - reflektiert wird, insbesondere abgegeben wird. Des Weiteren ist der Filterspiegel 13 derart ausgebildet, dass Strahlung im Rotlichtwellenlängenbereich von 600 bis 700 nm reflektierbar bzw. abgebbar ist. Zudem kann auch Strahlung im Wellenlängenbereich von oberhalb 700 nm reflektiert, insbesondere abgegeben, werden. Weitere Strahlung kann durch den Filterspiegel 13 ansonsten transmittiert und/oder absorbiert, insbesondere gefiltert, werden. Der Reflektionsgrad kann hierbei größer oder gleich 40%, bevorzugt zwischen 50 bis 70 %, betragen.

Des Weiteren zeigt Fig. 8, dass die Reflektoreinrichtung 10 eine Strahlung im Wellenlängenbereich von 100 bis 480 nm, vorzugsweise in einem Wellenlängenbereich von 280 bis 400 nm, und/oder in einem Wellenlängenbereich von 600 bis 700 nm und/oder im Wellenlängenbereich von größer 700 nm reflektiert, insbesondere abgibt. Der Reflektionsgrad kann hierbei größer oder gleich 40%, bevorzugt zwischen 50 bis 70%, betragen. Ansonsten kann die Reflektoreinrichtung 10 die Strahlung transmittieren und/oder absorbieren, insbesondere blocken. Demzufolge kann die Reflektoreinrichtung 10 und/oder der Reflektor 9 derart ausgebildet sein, dass lediglich Strahlung im UV-A- und/oder UV-B-Bereich und/oder Strahlung im Rotlichtbereich und/oder Infrarotstrahlung mit einer Wellenlänge von größer 700 nm über die Reflektoreinrichtung 10 abgegeben und/oder reflektiert wird.

Nicht dargestellt ist, dass der Reflektor 9 die erste und/oder zweite Beschichtung 5, 7 aufweisen, kann, so dass nur die gewünschte Wellenlängenbereiche der Strahlung reflektiert werden. Ferner ist nicht dargestellt, dass die Reflektoreinrichtung 10 ein Reflektionsmittel aufweisen kann, durch dass die von der Strahlungsquelle 2 ausgesendete Strahlung zumindest im Wesentlichen vollumfänglich auf den Reflektor 9 trifft.

Als Strahlungsquelle 2 kann eine Bestrahlungslampe, insbesondere eine Hochdruck- und/oder Höchstdruckgasentladungslampe, vorgesehen sein.

Die Fig. 1 und 2 zeigen ein Vorschaltgerät 14, das regelbar oder nicht regelbar ausgebildet sein kann. Die Strahlungsquelle 2 kann mittels des Vorschaltgeräts 14 dimmbar ausgebildet sein. Insbesondere kann ein magnetisches oder elektronisches Vorschaltgerät 14 vorgesehen sein.

### Bezugszeichenliste:

- 1: Bestrahlungsvorrichtung
- 2: Strahlungsquelle
- 3: Filtereinrichtung
- 4: Filterscheibe
- 5: erste Beschichtung
- 6: Vorderseite der Filterscheibe
- 7: zweite Beschichtung
- 8: Rückseite der Filterscheibe
- 9: Reflektor
- 10: Reflektoreinrichtung
- 11: Kühleinrichtung
- 12: Benutzer
- 13: Filterspiegel
- 14: Vorschaltgerät
- 15: Lampe
- 16: Gesichtsbereich
- 17: Liegefläche
- 18: Oberteil

## Patentansprüche

1. Bestrahlungsvorrichtung (1) zur Bestrahlung von menschlicher Haut, vorzugsweise des Gesichtes eines Benutzers (12), insbesondere zumindest mit Strahlung im Rotlichtbereich, mit einer Strahlungsquelle (2) und einer Filtereinrichtung (3) zur Filterung der von der Strahlungsquelle (2) abgestrahlten Strahlung,
wobei die Filtereinrichtung (3) derart ausgebildet ist,
dass die Bestrahlungsvorrichtung (1) Strahlung in einem Wellenlängenbereich von 280 bis 400 nm und Strahlung in einem Wellenlängenbereich von 600 bis 700 nm abgibt,
und wobei die Bestrahlungsvorrichtung (1) zumindest bereichsweise Strahlung in einem Wellenlängenbereich eines sichtbaren Farbtons des sichtbaren Lichtspektrums, insbesondere Strahlung im Grünlicht-, Rotlicht- und/oder Gelblichtbereich, abgibt, wobei die Filtereinrichtung (3) wenigstens eine Filterscheibe (4) aufweist, **gekennzeichnet dadurch, dass**:
die Filterscheibe (4) eine erste Beschichtung (5) aufweist,
wobei die erste Beschichtung (5) derart ausgebildet ist, dass UV-Strahlung in einem Wellenlängenbereich von 280 bis 400 nm und Strahlung im Rotlichtbereich in einem Wellenlängenbereich von 600 bis 700 nm und Strahlung mit einer Wellenlänge von größer als 700 nm transmittiert wird und wobei die erste Beschichtung (5) UV-Strahlung im UV-C-Bereich in einem Wellenlängenbereich von 100 bis 280 nm absorbiert,
wobei die erste Beschichtung (5) auf der der Strahlungsquelle zugewandten Vorderseite (6) der Filterscheibe (4) angeordnet ist, wobei die erste Beschichtung (5) als vollflächig ausgebildete Schicht vorgesehen ist,
die Filterscheibe (4) eine zweite Beschichtung (7) aufweist,
wobei die zweite Beschichtung (7) zur Filterung der Strahlung ausgebildet ist, wobei die Strahlung in wenigstens einen Wellenlängenbereich eines sichtbaren Farbtons des sichtbaren Lichtspektrums, transmittiert wird und, vorzugsweise, Strahlung mit einer Wellenlänge von größer 700 nm transmittiert wird, wobei ansonsten die zweite Beschichtung (7) reflektiert und/oder absorbiert,
wobei die zweite Beschichtung (7) als teilflächig ausgebildete Schicht ausgebildet ist.

2. Bestrahlungsvorrichtung nach Anspruch 1, **dadurch gekennzeichnet, dass** die Filtereinrichtung (3) derart ausgebildet ist, dass sie Strahlung mit einer Wellenlänge von unter 280 nm filtert.

3. Bestrahlungsvorrichtung nach einem der vorhergehenden Ansprüche, **dadurch gekennzeichnet, dass** der Transmissionsgrad der ersten Beschichtung (5) für UV-Strahlung in einem Wellenlängenbereich von 280 bis 400 nm und/oder Strahlung im Rotlichtbereich in einem Wellenlängenbereich von 600 bis 700 nm, vorzugsweise von 600 bis 650 nm, und/oder Strahlung in einer Wellenlänge von größer als 700 nm größer gleich 50%, bevorzugt zwischen 50 bis 100%, weiter bevorzugt zwischen 85 bis 98%, beträgt.

4. Bestrahlungsvorrichtung nach einem der vorhergehenden Ansprüche, **dadurch gekennzeichnet, dass** der Absorptionsgrad der ersten Beschichtung (5) für UV-Strahlung im UV-C-Bereich, vorzugsweise in einem Wellenlängenbereich von 100 bis 280 nm größer als 0,5, bevorzugt zwischen 0,6 und 1, weiter bevorzugt zwischen 0,8 bis 0,99, beträgt.

5. Bestrahlungsvorrichtung nach einem der vorhergehenden Ansprüche, **dadurch gekennzeichnet, dass** die erste Beschichtung (5) derart ausgebildet ist, dass UV-B-Strahlung in einem Wellenlängenbereich von 280 bis 320 nm mit einem Transmissionsgrad von kleiner gleich 50%, bevorzugt zwischen 0,1 bis 50%, weiter bevorzugt zwischen 1 bis 8%, transmittiert wird.

6. Bestrahlungsvorrichtung nach einem der vorhergehenden Ansprüche, **dadurch gekennzeichnet, dass** die erste Beschichtung (5) derart ausgebildet ist, dass UV-Strahlung in einem Wellenlängenbereich von 320 bis 400 nm mit einem Transmissionsgrad von größer gleich 40%, bevorzugt zwischen 45 bis 95%, weiter bevorzugt zwischen 50 bis 70%, transmittiert wird.

7. Bestrahlungsvorrichtung nach einem der vorhergehenden Ansprüche, **dadurch gekennzeichnet, dass** die erste Beschichtung (5) derart ausgebildet ist, dass Strahlung im Blaulichtbereich in einem Wellenlängenbereich von 400 bis 480 nm mit einem Transmissionsgrad von größer gleich 20%, bevorzugt zwischen 30 bis 95%, weiter bevorzugt zwischen 60 bis 80%, transmittiert wird.

8. Bestrahlungsvorrichtung nach einem der vorhergehenden Ansprüche, **dadurch gekennzeichnet, dass** die zweite Beschichtung (7) auf der ersten Beschichtung (5) angeordnet ist, insbesondere fest mit der ersten Beschichtung (5) verbunden ist, und/oder dass die zweite Beschichtung (7) auf der Rückseite (8) der Filterscheibe (4), der Strahlungsquelle (2) abgewandt, angeordnet, insbesondere aufgebracht, ist.

9. Bestrahlungsvorrichtung nach einem der vorhergehenden Ansprüche, **dadurch gekennzeichnet, dass** die zweite Beschichtung (7) derart ausgebildet ist, dass UV-Strahlung in einem Wellenlängenbereich von 100 bis 400 nm und/oder Strahlung im Blaulichtbereich in einem Wellenlängenbereich von 400 bis 480 nm mit einem Transmissionsgrad von kleiner gleich 20%, bevorzugt zwischen 0,01 bis 50%, weiter bevorzugt zwischen 0,1 bis 5%, transmittiert wird.

10. Bestrahlungsvorrichtung nach einem der vorhergehenden Ansprüche, **dadurch gekennzeichnet, dass** die zweite Beschichtung (7) derart ausgebildet ist, dass Strahlung im Rotlichtbereich in einem Wellenlängenbereich von 600 bis 700 nm und/oder Strahlung mit einer Wellenlänge von größer als 700 nm mit einem Transmissionsgrad von größer gleich 50%, bevorzugt zwischen 50 bis 100%, weiter bevorzugt zwischen 75 bis 85%, transmittiert wird.

11. Bestrahlungsvorrichtung nach einem der vorhergehenden Ansprüche, **dadurch gekennzeichnet, dass** die zweite Beschichtung (7) derart ausgebildet ist, dass UV-Strahlung in einem Wellenlängenbereich von 320 bis 400 nm mit einem Transmissionsgrad von größer gleich 40%, bevorzugt zwischen 45 bis 95%, weiter bevorzugt zwischen 50 bis 70%, transmittiert wird.

12. Bestrahlungsvorrichtung nach einem der vorhergehenden Ansprüche, **dadurch gekennzeichnet, dass** wenigstens eine einen Reflektor (9) aufweisende Reflektoreinrichtung (10) und, vorzugsweise, eine der Reflektoreinrichtung (10) zugeordnete Kühleinrichtung (11) vorgesehen sind, wobei die Strahlungsquelle (2) im Bereich des Reflektors (9) angeordnet ist.

13. Bestrahlungsvorrichtung nach Anspruch 12, **dadurch gekennzeichnet, dass** die Reflektoreinrichtung (10) eine Strahlung im Wellenlängenbereich von 100 bis 480 nm, vorzugsweise in einem Wellenlängenbereich von 280 bis 400 nm, und/oder in einem Wellenlängenbereich von 600 bis 700 nm und/oder im Wellenlängenbereich von größer 700 nm reflektiert wird, vorzugsweise mit einem Reflektionsgrad von größer gleich 40%, bevorzugt zwischen 45 bis 95%, weiter bevorzugt zwischen 50 bis 70%, insbesondere wobei die Reflektoreinrichtung (10) ansonsten transmittiert und/oder absorbiert.

## Claims

1. Radiation apparatus (1) for the radiation of human skin, preferably the face of a user (12), in particular at least with radiation in the red light range, with a radiation source (2) and a filtering device (3) for filtering the radiation emitted by the radiation source (2),
wherein the filtering device (3) is designed in such a way
that the radiation apparatus (1) emits radiation in a wavelength range from 280 to 400 nm and radiation in a wavelength range from 600 to 700 nm,
and
wherein the radiation apparatus (1) emits, at least in areas, radiation in a wavelength range of a visible color shade of the visible light spectrum, in particular radiation in the green light, red light and/or yellow light range,
wherein the filtering device (3) comprises at least one filter disk (4),
**characterized in that**
the filter disk (4) comprises a first coating (5),
wherein the first coating (5) is designed in such a way UV radiation in a wavelength range from 280 to 400 nm and radiation in the red light range in a wavelength range from 600 to 700 nm and radiation with a wavelength greater than 700 nm is transmitted and wherein the first coating (5) absorbs UV radiation in the UV-C range in a wavelength range from 100 to 280 nm,
wherein the first coating (5) is arranged on the front side (6) of the filter disk (4) facing the radiation source, wherein the first coating (5) is provided as a layer with a full surface area,
the filter disk (4) comprises a second coating (7),
wherein the second coating (7) is designed for filtering the radiation, wherein the radiation is transmitted in at least one wavelength range of a visible color shade of the visible light spectrum and, preferably, radiation with a wavelength greater than 700 nm is transmitted, wherein otherwise the second coating (7) reflects and/or absorbs,
wherein the second coating (7) is designed as a layer with a partial surface area.

2. Radiation apparatus according to claim 1, **characterized in that** the filtering device (3) is designed in such a way that it filters radiation with a wavelength of less than 280 nm.

3. Radiation apparatus according to one of the preceding claims, **characterized in that** the transmittance of the first coating (5) for UV radiation in a wavelength range from 280 to 400 nm and/or radiation in the red light range in a wavelength range from 600 to 700 nm, preferably from 600 to 650 nm, and/or radiation in a wavelength greater than 700 nm is greater than or equal to 50%, preferably between 50 to 100%, more preferably between 85 to 98%.

4. Radiation apparatus according to one of the preceding claims, **characterized in that** the degree of absorption of the first coating (5) for UV radiation in the UV-C range, preferably in a wavelength range from 100 to 280 nm, is greater than 0.5, preferably between 0.6 and 1, more preferably between 0.8 and 0.99.

5. Radiation apparatus according to one of the preceding claims, **characterized in that** the first coating (5) is designed in such a way that UV-B radiation in a wavelength range from 280 to 320 nm is transmitted with a transmittance of less than or equal to 50%, preferably between 0.1 to 50%, more preferably between 1 to 8%.

6. Radiation apparatus according to one of the preceding claims, **characterized in that** the first coating (5) is designed in such a way that UV radiation is transmitted in a wavelength range of 320 to 400 nm with a transmittance of greater than or equal to 40%, preferably between 45 to 95%, more preferably between 50 to 70%.

7. Radiation apparatus according to one of the preceding claims, **characterized in that** the first coating (5) is designed in such a way that radiation in the blue light range in a wavelength range from 400 to 480 nm is transmitted with a transmittance of greater or equal 20%, preferably between 30 to 95%, more preferably between 60 to 80%.

8. Radiation apparatus according to one of the preceding claims, **characterized in that** the second coating (7) is arranged on the first coating (5), in particular is firmly connected to the first coating (5), and/or **in that** the second coating (7) is arranged, in particular applied, on the rear side (8) of the filter disk (4), facing away from the radiation source (2).

9. Radiation apparatus according to one of the preceding claims, **characterized in that** the second coating (7) is designed in such a way that UV radiation in a wavelength range from 100 to 400 nm and/or radiation in the blue light range in a wavelength range from 400 to 480 nm is transmitted with a transmittance of less than or equal to 20%, preferably between 0.01 to 50%, more preferably between 0.1 to 5%.

10. Radiation apparatus according to one of the preceding claims, **characterized in that** the second coating (7) is designed in such a way radiation in the red light range in a wavelength range from 600 to 700 nm and/or radiation with a wavelength greater than 700 nm with a transmittance greater than or equal to 50%, preferably between 50 to 100%, more preferably between 75 to 85%.

11. Radiation apparatus according to one of the preceding claims, **characterized in that** the second coating (7) is designed in such a way that UV radiation is transmitted in a wavelength range of 320 to 400 nm with a transmittance of greater than or equal to 40%, preferably between 45 to 95%, more preferably between 50 to 70%.

12. Radiation apparatus according to one of the preceding claims, **characterized in that** at least one reflector device (10) comprising a reflector (9) and, preferably, a cooling device (11) associated with the reflector device (10) are provided, wherein the radiation source (2) is arranged in the region of the reflector (9).

13. Radiation apparatus according to claim 12, **characterized in that** the reflector device (10) reflects a radiation in a wavelength range from 100 to 480 nm, preferably in a wavelength range from 280 to 400 nm, and/or in a wavelength range from 600 to 700 nm and/or in a wavelength range greater than 700 nm, preferably with a degree of reflection of greater than or equal to 40%, preferably between 45 and 95%, more preferably between 50 and 70%, in particular wherein the reflector device (10) otherwise transmits and/or absorbs.

## Revendications

1. Appareil d'irradiation (1) pour irradier la peau humaine, de préférence le visage d'un utilisateur (12), en particulier au moins avec un rayonnement dans la gamme de la lumière rouge, avec une source de rayonnement (2) et un dispositif de filtrage (3) pour filtrer le rayonnement émis par la source de rayonnement (2),
dans lequel le dispositif de filtrage (3) est formé de telle manière,
en ce que l'appareil d'irradiation (1) émet un rayonnement dans une gamme de longueurs d'onde de 280 à 400 nm et un rayonnement dans une gamme de longueurs d'onde de 600 à 700 nm,
et
dans lequel l'appareil d'irradiation (1) émet, au moins par zones, un rayonnement dans une gamme de longueurs d'onde d'une teinte visible du spectre de la lumière visible, en particulier un rayonnement dans la gamme de la lumière verte, rouge et/ou jaune,
le dispositif de filtrage (3) comprenant au moins un disque filtrant (4),
**caractérisé en ce que**
le disque filtrant (4) comporte un premier revêtement (5),
le premier revêtement (5) étant conçu de manière à transmettre le rayonnement UV dans une gamme de longueurs d'onde de 280 à 400 nm et le rayonnement dans la gamme de lumière rouge dans une gamme de longueurs d'onde de 600 à 700 nm et le rayonnement d'une longueur d'onde supérieure à 700 nm, et le premier revêtement (5) absorbant le rayonnement UV dans la gamme UV-C dans une gamme de longueurs d'onde de 100 à 280 nm,
le premier revêtement (5) étant disposé sur la face frontale (6) du disque filtrant (4) tournée vers la source de rayonnement, le premier revêtement (5) étant prévu sous la forme d'une couche formée sur toute la surface,
le disque filtrant (4) comporte un second revêtement (7),
le second revêtement (7) étant adapté pour filtrer le rayonnement, le rayonnement étant transmis dans au moins une gamme de longueurs d'onde d'une teinte visible du spectre de la lumière visible et, de préférence, un rayonnement d'une longueur d'onde supérieure à 700 nm étant transmis, sinon le second revêtement (7) réfléchit et/ou absorbe,
le second revêtement (7) étant constitué d'une couche formée sur une partie de la surface.

2. Appareil d'irradiation selon la revendication 1, **caractérisé en ce que** le dispositif de filtrage (3) est conçu de telle manière qu'il filtre le rayonnement d'une longueur d'onde inférieure à 280 nm.

3. Appareil d'irradiation selon l'une des revendications précédentes, **caractérisé en ce que** la transmittance du premier revêtement (5) pour le rayonnement UV dans une gamme de longueurs d'onde de 280 à 400 nm et/ou le rayonnement dans la gamme de lumière rouge dans une gamme de longueurs d'onde de 600 à 700 nm, de préférence de 600 à 650 nm, et/ou le rayonnement dans une longueur d'onde supérieure à 700 nm est supérieure ou égale à 50 %, de préférence entre 50 et 100 %, plus préférablement entre 85 et 98 %.

4. Appareil d'irradiation selon l'une des revendications précédentes, **caractérisé en ce que** le coefficient d'absorption du premier revêtement (5) pour le rayonnement UV dans la gamme UV-C, de préférence dans une gamme de longueurs d'onde de 100 à 280 nm, est supérieur à 0,5, de préférence entre 0,6 et 1, plus préférablement entre 0,8 et 0,99.

5. Appareil d'irradiation selon l'une des revendications précédentes, **caractérisé en ce que** le premier revêtement (5) est conçu de telle manière que le rayonnement UV-B dans une gamme de longueurs d'onde de 280 à 320 nm est transmis avec une transmittance inférieure ou égale à 50 %, de préférence entre 0,1 et 50 %, plus préférablement entre 1 et 8 %.

6. Appareil d'irradiation selon l'une des revendications précédentes, **caractérisé en ce que** le premier revêtement (5) est conçu de telle manière que le rayonnement UV dans une gamme de longueurs d'onde de 320 à 400 nm est transmis avec une transmittance supérieure ou égale à 40 %, de préférence entre 45 et 95 %, plus préférablement entre 50 et 70 %.

7. Appareil d'irradiation selon l'une des revendications précédentes, **caractérisé en ce que** le premier revêtement (5) est conçu de telle manière que le rayonnement dans la gamme de la lumière bleue dans une gamme de longueurs d'onde de 400 à 480 nm est transmis avec une transmittance supérieure ou égale à 20%, de préférence entre 30 et 95%, plus préférablement entre 60 et 80%.

8. Appareil d'irradiation selon l'une des revendications précédentes, **caractérisé en ce que** le second revêtement (7) est disposé sur le premier revêtement (5), en particulier est solidement relié au premier revêtement (5), et/ou **en ce que** le second revêtement (7) est disposé, en particulier appliqué, sur la face arrière (8) du disque filtrant (4), opposée à la source de rayonnement (2).

9. Appareil d'irradiation selon l'une des revendications précédentes, **caractérisé en ce que** le second revêtement (7) est conçu de telle manière que le rayonnement UV dans une gamme de longueur d'onde de 100 à 400 nm et/ou le rayonnement dans la gamme de lumière bleue dans une gamme de longueur d'onde de 400 à 480 nm est transmis avec une transmittance inférieure ou égale à 20 %, de préférence entre 0,01 et 50 %, plus préférablement entre 0,1 et 5 %.

10. Appareil d'irradiation selon l'une des revendications précédentes, **caractérisé en ce que** le second revêtement (7) est conçu de telle manière que le rayonnement dans le domaine de la lumière rouge dans une gamme de longueur d'onde de 600 à 700 nm et/ou le rayonnement avec une longueur d'onde supérieure à 700 nm est transmis avec une transmittance supérieure ou égale à 50 %, de préférence entre 50 et 100 %, plus préférablement entre 75 et 85 %.

11. Appareil d'irradiation selon l'une des revendications précédentes, **caractérisé en ce que** le second revêtement (7) est conçu de telle manière que le rayonnement UV dans une gamme de longueurs d'onde de 320 à 400 nm est transmis avec une transmittance supérieure ou égale à 40 %, de préférence entre 45 et 95 %, plus préférablement entre 50 et 70 %.

12. Appareil d'irradiation selon l'une des revendications précédentes, **caractérisé en ce qu'**il est prévu au moins un dispositif réflecteur (10) comprenant un réflecteur (9) et, de préférence, un dispositif de refroidissement (11) associé au dispositif réflecteur (10), la source de rayonnement (2) étant disposée dans la région du réflecteur (9).

13. Appareil d'irradiation selon la revendication 12, **caractérisé en ce que** le dispositif réflecteur (10) réfléchit le rayonnement dans la gamme de longueurs d'onde de 100 à 480 nm, de préférence dans une gamme de longueurs d'onde de 280 à 400 nm, et/ou dans une gamme de longueurs d'onde de 600 à 700 nm et/ou dans la gamme de longueurs d'onde supérieure à 700 nm, de préférence avec un degré de réflexion supérieur ou égal à 40 %, de préférence entre 45 et 95 %, plus préférablement entre 50 et 70 %, en particulier le dispositif réflecteur (10) transmettant et/ou absorbant autrement.
